# EUROPEAN PATENT SPECIFICATION

(11) **EP 1 574 194 B1**
(45) Date of publication and mention of the grant of the patent: **13.01.2010**
(21) Application number: 04005733.3
(22) Date of filing: 10.03.2004
(51) Int. Cl.: A61F 13/551

(54) **Process and apparatus for making individually packaged disposable absorbent articles**
Verfahren und Apparat zur Herstellung von einzeln verpackten, absorbierenden Wegwerfartikeln
Procédé et appareil de manufacture pour articles absorbants, jetables en conditionnement individuel

(43) Date of publication of application: 14.09.2005
(73) Proprietor: THE PROCTER & GAMBLE COMPANY, Cincinnati, Ohio 45202 (US)
(72) Inventor: Cesiro, Luca, 65126 Pescara (IT); Wirnshofer, Andreas, 92539 Schonsee (DE); Breda, Sandro, 65020 Turrivalignani (Pescara) (IT); Branca, Andrea, 65124 Pescara (IT)
(74) Representative: Veronese, Pancrazio

(56) References cited:
- EP-A- 0 386 816
- US-A- 3 445 897
- US-A- 4 556 146
- US-A1- 2002 092 604
- US-B1- 6 531 027

## Description

### Field of the Invention

The present invention relates to disposable absorbent articles, particularly catamenial pads and pantiliners, which are individually packaged prior to use. Still more particularly, this invention relates to a process and an apparatus for making the individually packaged disposable absorbent articles.

### Background of the Invention

Disposable absorbent articles are well known in the prior art and have many uses. For example, disposable diapers are intended to absorb and retain urine; bandages are intended to absorb and retain blood and other body exudates; while catamenial pads are intended to absorb and retain menstrual fluids. In each instance, the disposable absorbent article absorbs and retains a liquid, thereby preventing the liquid from soiling, wetting, or otherwise contaminating the vicinity surrounding the point of liquid discharge.

In general, disposable absorbent articles all have the same basic structure which comprises an absorbent core comprised between a liquid permeable, user-contacting topsheet layer which permits liquid to penetrate its thickness and contact the absorbent core where the liquid is retained, and a liquid impervious, garment contacting backsheet element. The prior art teaches numerous variations of and elements in addition to the basic absorbent core, topsheet and backsheet arrangement, with each variation or additional element being directed to improving a specific characteristic of the disposable absorbent article.

While there are a great many variations in the specific structural features of disposable absorbent articles, they are frequently presented to the consumer in the same manner. Essentially, the disposable absorbent article, irrespective of what specific structural features are used, is packaged in a box or bag from which the consumer withdraws the article as needed.

The problem of protecting the disposable absorbent article once it is removed from the package in which it is sold has been addressed in the prior art. Catamenial pads, for example, are commonly packaged individually and sold to the consumer in some sort of container, typically a bag or box, which holds a convenient number of the individually packaged articles. For example, in patent application WO 94/14396, entitled Fold and Wrap Package for Catamenial Pads Providing Convenient Disposal Means, an individual package for a catamenial pad is disclosed which provides containment prior to use and also convenient disposal of a soiled catamenial pad after use. The individual package comprises a wrapper configured to have a pouch and a closure flap. The pouch is designed so that an unused catamenial pad may be disposed within the pouch, typically in folded configuration, with the flap being positioned over the mouth of the pouch, and frangibly bonded thereto e.g. with an adhesive tape tab in order to close the pouch prior to use. The package is used by peeling the flap away from the pouch by breaking the frangible bonds between the flap and the pouch, removing the unused catamenial pad from the pouch, and finally completely opening the pouch such that the package is a flat wrapper. A soiled catamenial pad can be then secured to the flat wrapper typically by means of its panty fastening adhesive, the catamenial pad and the wrapper arranged into a disposal configuration, typically rolled and folded on themselves, and finally secured in this configuration by securing the adhesive tape tab to another portion of the wrapper.

US Patent Number 4,556,146 entitled Individually Packaged Disposable Absorbent Article discloses a disposable absorbent article, such as a catamenial pad, associated with a wrapper which overlays one major surface of the catamenial pad, and is releasably attached thereto, typically by means of the catamenial pad panty fastening adhesive. The wrapper is larger than the external perimeter of the disposable absorbent article, so that when the disposable absorbent article and the wrapper are folded as a unit, typically around two fold-axes, the respective longitudinal side flaps of the wrapper may be frangibly sealed together along common longitudinal edges, thereby providing the disposable absorbent article with an individual package. Preferably, the disposable absorbent article is releasably attached directly to the wrapper by means of its panty fastening adhesive element, thereby eliminating the need to provide a release paper.

Typically the panty fastening adhesive, as disclosed in US 4,556,146, is provided as a coating of a known pressure sensitive adhesive in strips or other suitable patterns, such as for example as a single strip oriented longitudinally and substantially covering the centre of the catamenial pad backsheet.

From a process point of view, the adhesive coating can be actually applied to the release paper, and then transferred to the backsheet of the disposable absorbent article by joining the release paper in the desired position to the backsheet, before formation of the individual package, which can be performed either according to WO 94/14396, or according to US 4,556,146, both documents as mentioned above. In the latter case, the release paper has to be permanently attached to the wrapper material before formation of the individual package. Alternatively, direct application of the adhesive coating onto the backsheet in the desired pattern can be provided, with subsequent joining of the absorbent article to the wrapper material, which eliminates the need of a separate release paper, simplifies the process and saves material. This method is particularly suitable for the individually packaged disposable absorbent article according to US 4,556,146.

Direct adhesive application onto the backsheet usually occurs onto a continuous web-like layered structure from which the individual absorbent articles are subsequently formed by cutting them from said structure along a respective desired outer perimeter. The layered structure can already comprise all the elements, namely layers, which eventually form the disposable absorbent article, or some further elements can be added in a subsequent step after the cutting step.

It has been discovered that in disposable absorbent articles, particularly of the thin type such as thin sanitary napkins and pantiliners, the coverage of the panty fastening adhesive onto the backsheet should be preferably extended substantially to the whole surface of the backsheet intended to get in contact with the undergarment in use, or at least to portions of the outer perimeter of the article, preferably those portions extending along the longitudinal edges of the disposable absorbent article. This is advantageous in terms of stability during use of the article, which in turn is capable of staying in place in the crotch area of the undergarment, where it has been applied by the user, more effectively withstanding the stresses and tensions induced by the user's movements.

However in an individually packaged disposable absorbent article, namely a catamenial pad or pantiliner, the preferred features of a panty fastening adhesive coverage at least partially extending up to the outer perimeter of the absorbent article, and the absence of the release paper, constitute in combination two contrasting, and so far antithetical needs from a process point of view. On one hand in fact the absence of a release paper necessarily implies direct adhesive coating onto the backsheet. On the other hand, according to the known technique, a patterned coating of adhesive which is exactly registered with at least part of the outer perimeter of a disposable absorbent article is not practically feasible, either when the coating is applied before or when is applied after the single disposable absorbent article has been formed from a continuous web-like structure by cutting it along the desired outer perimeter. The only theoretical alternative is to apply the adhesive coating to the continuous web-like structure in such a way that it overlaps the portions of the outer perimeter of the disposable absorbent articles along which the adhesive is meant to arrive, and that the subsequent cutting operation in order to form and separate the individual articles occurs directly through the adhesive layer. As it is known in the art, this leads to adhesive sticking to the knife implement, with obvious problems of adhesive build up on the knife and unreliability of the process.

It has been surprisingly discovered that the problem of providing in a reliable and simple way individually wrapped disposable absorbent articles where each article is releasably attached to the wrapper element without the need of a release paper, and moreover where the panty fastening adhesive extends along at least part of the outer perimeter of each individual disposable absorbent article, can be solved by combining in a process for providing release paper-free, individually wrapped disposable absorbent articles, the direct application of the adhesive for panty fastening onto the backsheet layer in a continuous web-like layered structure from which the individual disposable absorbent articles are to be formed, and the cutting of the individual disposable absorbent articles from the continuous web-like layered structure at least partially through the adhesive layer, at least along the portions of the outer perimeter where the adhesive must be present. Direct cut through the adhesive layer without adhesive sticking and build up on the cutting implement is done by applying the technique of cutting with a knife which is internally cooled.

According to the present invention, a method and an apparatus are described for providing individually wrapped, release paper-free disposable absorbent articles having the panty fastening adhesive extending along at least part of the respective outer perimeter.

### Summary of the Invention

The present invention relates to a process for manufacturing individually packaged disposable absorbent articles comprising the steps of:
providing a continuous web-like layered structure comprising at least a liquid pervious outer structure, a liquid impervious outer structure, and an absorbent material intermediate the liquid pervious outer structure and the liquid impervious outer structure,
applying a layer of pressure sensitive adhesive for garment attachment onto at least part of the liquid impervious outer structure,
cutting individual disposable absorbent articles along a perimeter from the continuous web-like layered structure, wherein the cutting occurs at least partially directly through the layer of pressure sensitive adhesive, and wherein the perimeter of each of the individual disposable absorbent articles has longitudinal perimeter segments substantially parallel to a longitudinal axis of the individual disposable absorbent articles; wherein the cutting step is carried out with a cutting implement comprising at least one knife which is internally cooled;
releasably attaching each of the individual disposable absorbent articles to a respective wrapper element by means of the layer of pressure sensitive adhesive;
folding the wrapper element around each of the individual disposable absorbent articles;
sealing the wrapper element around at least part of the perimeter of each of the individual disposable absorbent articles in order to form individually packaged disposable absorbent articles.

The present invention also relates to an apparatus for performing the process above, comprising a cutting implement for the cutting of the individual disposable absorbent articles from the continuous web-like layered structure, which comprises at least one knife which is internally cooled.

### Brief Description of the Drawings

Figure 1 shows a continuous, web-like layered structure from which the disposable absorbent articles are formed.
Figure 2 shows a disposable absorbent article releasably attached to a wrapper element.
Figure 3 shows a disposable absorbent article and a wrapper element partially folded as a unit around a first fold-axis, and just before being folded around a second fold-axis and sealed in order to form an individually wrapped disposable absorbent article.
Figure 4 shows a scheme for illustrating the key components of the process and apparatus of the present invention.

### Detailed Description of the Invention

The present invention refers to a process and to an apparatus for providing individually packaged disposable absorbent articles. As used herein the term "absorbent article" refers to those articles intended to absorb and retain liquid and in particular those articles which are placed against or in proximity to a wearer's body to absorb and contain the various liquids discharged from the body, e.g. perspiration, blood, menses, urine, vaginal secretions and the like. A "disposable absorbent article" is an absorbent article which is intended to be discarded after a single use, i.e. which is not intended to be laundered or otherwise restored and reused. Particularly, the disposable absorbent article is described below by reference to a sanitary napkin or pantiliner.

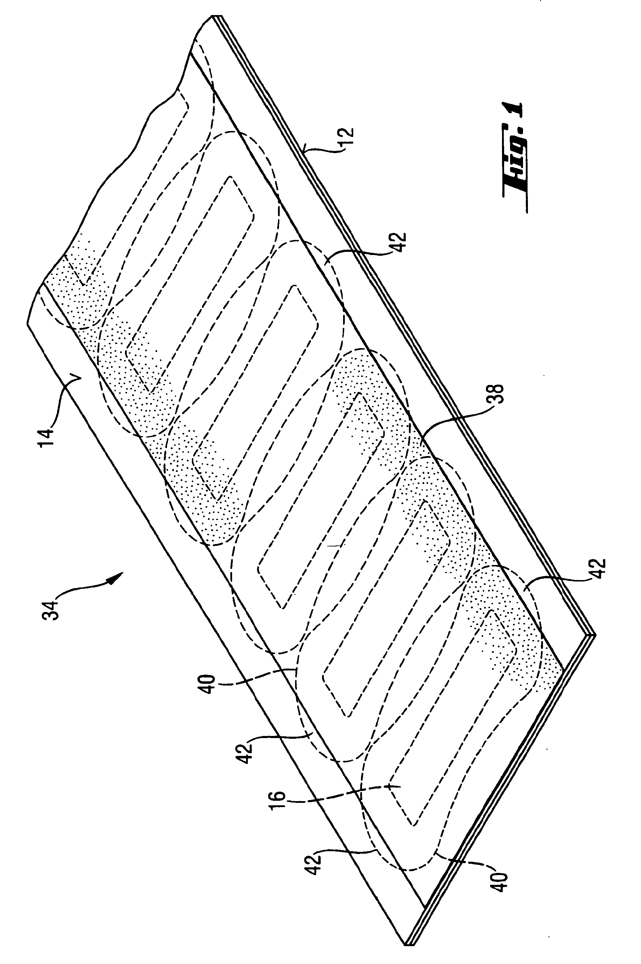

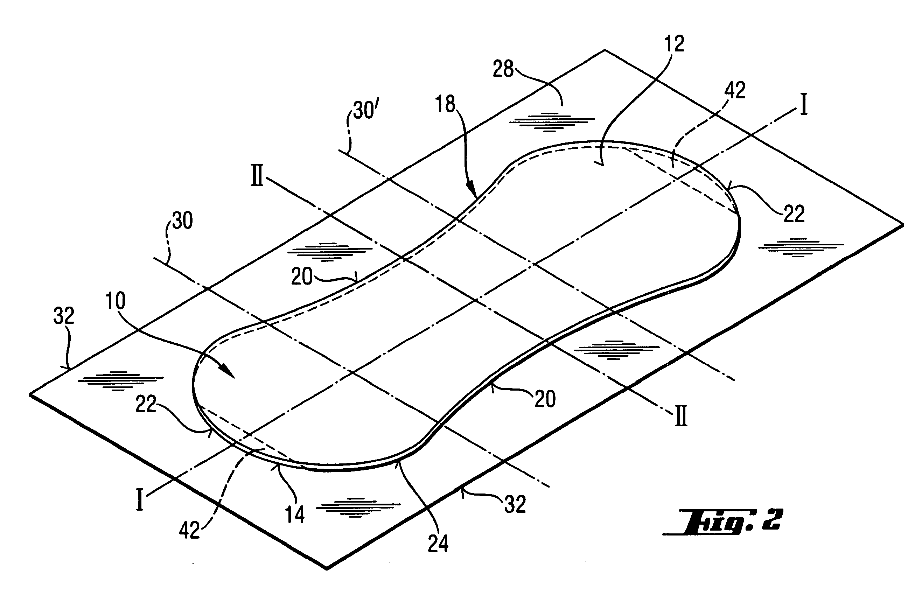

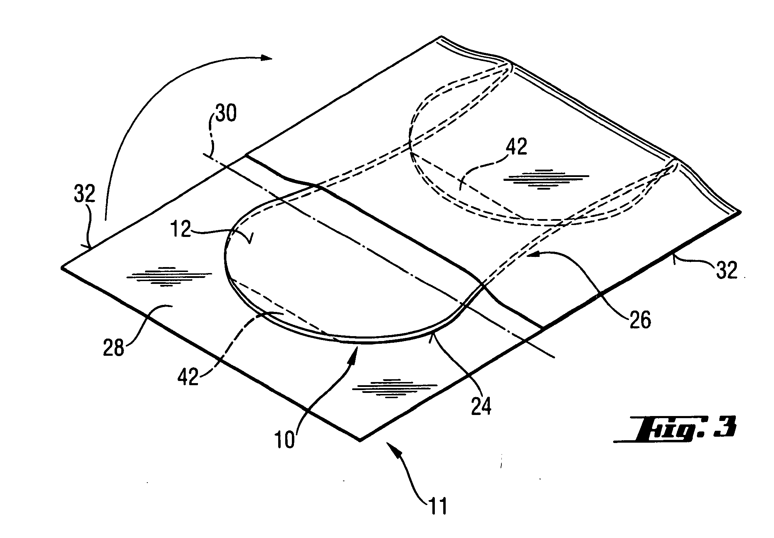

As shown in Figures 2 and 3, the disposable absorbent article 10 referred to in the present invention has a body facing surface, typically provided by a liquid pervious outer structure 12 usually termed topsheet; a garment facing surface, typically provided by a liquid impervious outer structure 14, which is preferably also water vapour and/or gas permeable, usually termed backsheet; and an absorbent structure 16 (not shown in Figures 2 and 3 for ease of representation, but indicated in transparency in Figure 1) placed between the body facing surface and the garment facing surface, usually termed the absorbent core. The absorbent article has a longitudinal axis I-I and a transverse axis II-II, perpendicular to each other, and is delimited by a perimeter 18 having longitudinal perimeter segments 20, corresponding to the portions of the perimeter 18 which are substantially parallel to the longitudinal axis, and transverse perimeter segments 22, corresponding to the portions of the perimeter 18 which are substantially parallel to the transverse axis. The absorbent article 10 can also comprise any of the components or features usual in the art, for example, it can comprise further layers and elements in addition to those mentioned above. The absorbent article 10, typically a sanitary napkin or pantiliner, is also provided with an adhesive element 24 on the garment facing surface, typically on the backsheet 14 which, in use, serves to affix the absorbent article 10 to the wearer's undergarment thereby maintaining the article in place against the wearer's body. The adhesive element 24 is typically a layer of a pressure sensitive adhesive. In the disposable absorbent article 10 the adhesive element 24 is covered by, and adhered thereto, a wrapper element 28 prior to use, which forms a pouch or bag 26, thereby providing the individually packaged disposable absorbent article 11 referred to in the present invention.

In order to more fully assess the utility of the process and apparatus of the present invention a description of a typical disposable absorbent article follows.

### Topsheet

The topsheet is compliant, soft feeling, and non-irritating to the wearer's skin. Useful topsheets are well known in the art and may be manufactured from either hydrophobic or hydrophilic fibres and may, for example, be cared, spun bonded, melt blown, or air laid. Alternatively, the topsheet may be a continuous film or sheet of, for example, thermoplastic material which is apertured. When referring to the topsheet a multi layer structure or a monolayer structure is contemplated.

### Absorbent structure

When referring to the absorbent structure a multi layer structure or a mono layer structure are contemplated. Useful absorbent structures are well-known in the art and may be manufactured from a wide variety of materials which are capable of absorbing and retaining liquids. For example, a batt of absorbent fibres, a multiplicity of plies of creped cellulose wadding, or any equivalent material may be used. Known absorbent gelling materials are also typically comprised in the absorbent structure. The absorbent capacity of the material used must be sufficient to absorb and retain the expected liquid loading in the intended use of the article without undue bulk. In a preferred embodiment the absorbent structure is a thin layered structure comprising two or more fibrous layers and particles of absorbent gelling materials comprised therebetween.

### Backsheet

The backsheet primarily prevents the exudates absorbed and contained in the absorbent structure from wetting articles that contact the absorbent product such as underpants, pants, pajamas and undergarments. The backsheet may be manufactured from any flexible, liquid impermeable material which is non irritating to the wearer. Typically, the backsheet is a sheet of polyethylene film. Preferably the backsheet is also water vapour or gas permeable. When referring to the backsheet a multi layer structure or a mono layer structure is contemplated.

### The panty-fastening adhesive

The backsheet typically forms the garment-facing surface of the absorbent article on which the panty fastening adhesive is placed. Panty-fastening adhesives can comprise any adhesive or glue used in the art for such purposes. These adhesives typically are pressure sensitive and remain tacky well below their application temperature. Suitable adhesives are for example Savare LA203 and Savare LA303 made by Savare I.C. of Milan in Italy, Coramelt 867 by Koemmerling in Pirmasens in Germany, Fuller D3964ZP and Fuller H-2238ZP manufactured by the H.B. Fuller Co. in Lueneburg, in Germany, NS34-2823 as manufactured by National Starch and Chemical of Bridgewater, N.J.

In accordance with the teaching of this invention, and with specific reference to Figures 2 and 3, the disposable absorbent article 10 is individually packaged into a bag or pouch 26 prior to use. The bag 26 is made of a wrapper element 28, typically a plastic film, which typically completely encases the disposable absorbent article 10 and can be opened by the user in order to take the absorbent article and wear it. The absorbent article 10 is releasably affixed to the wrapper element 28 by means of the panty-fastening adhesive, namely the adhesive element 24, therefore providing the advantage of protecting the panty-fastening adhesive until it is used for attaching the absorbent article to the undergarment, without the need of a separate release paper. According to a preferred embodiment of the present invention, the individually packaged disposable absorbent article 11 is configured according to patent US 4,556,146, namely as illustrated in Figures 1 and 2 therein, where no separate release paper is used, but the disposable absorbent article 10 is directly affixed to the wrapper element by means of the pressure-sensitive panty-fastening adhesive. The individual package can be generally formed by folding the wrapper element 28 around the individual disposable absorbent article 10, and by sealing it around at least part of the perimeter of the disposable absorbent article 10. More preferably, according to the preferred embodiment of the present invention disclosed in US 4,566,146, the individual package (bag or pouch 26) of the disposable absorbent article 10 is made by folding the wrapper element 28 and the affixed absorbent article 10 as a unit, i.e. they are folded together with the absorbent article 10 remaining in place with respect to the wrapper element 28. Preferably, the absorbent article 10 and the wrapper element 28 are folded lengthwise into thirds about two fold-axes 30, 30' substantially parallel to the transverse axis II-II of the absorbent article 10. The wrapper element 28 overlays the backsheet 14 of the absorbent article 10 and preferably and typically has longitudinal side portions 32 extending beyond the longitudinal perimeter segments 22 of the absorbent article. The respective longitudinal side portions 32 of the wrapper element 28 are frangibly sealed together along common longitudinal edges using any of the well-known sealing techniques, such as for example heat sealing, ultrasonic sealing, crimping, in order to form the individual package.

Figure 3 shows a disposable absorbent article 10 and the wrapper element 28 partially folded around the first fold axis 30', and just before the last third of the absorbent article 10 and wrapper element 28 are folded as a unit around the second fold line 30, in the direction indicated by the arrow.

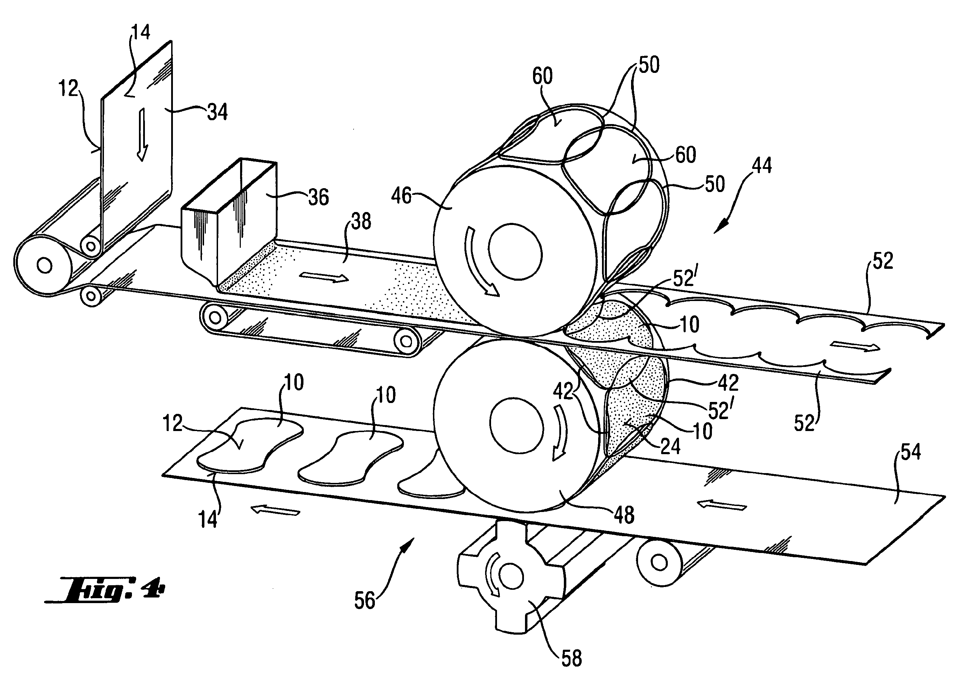

Figure 4 schematically illustrates a process and an apparatus according to the present invention for making individually packaged disposable absorbent articles. A continuous web-like layered structure 34, from which according to a preferred embodiment of the present invention the disposable absorbent articles 10 are obtained by cutting the structure around a predetermined contour in order to get the desired shape, is provided and conveyed in the machine direction indicated with an arrow following a designated transport path. The continuous web-like layered structure 34 is illustrated more in detail in Figure 1, which shows the basic components of said structure. The continuous web-like layered structure 34 typically comprises as individual layers the elements which will eventually form the disposable absorbent article 10, and which will be identified in Figure 1 with the same numerals as shown in the absorbent article of Figure 2, and with the same denominations, with the proviso that an element identified with a same numeral will correspond to a single element of the finished disposable absorbent article 10 in Figure 2, and to a typically continuous layer or structure in Figure 1, from which the individual absorbent articles are obtained by cutting. This of course does not exclude that certain elements, such as for example the absorbent core as illustrated in Figure 1, can be alternatively provided according to certain embodiments as discrete elements already in the continuous web-like layered structure of Figure 1. Figure 1 in particular also shows for clarity the predetermined contour along which the individual disposable absorbent articles are finally cut from the continuous web-like layered structure, indicated as a dotted line 40, which in turn corresponds to the desired shape for the individual disposable absorbent articles. Said shape is an hourglass shape, as illustrated in Figures 1 to 4, but can of course be varied as it is known in the art.

The continuous web-like layered structure 34 of Figure 1 comprises a liquid pervious outer layer 12, intended to form the topsheet of the finished article, a liquid impervious outer layer 14, intended to form the backsheet in the finished article, and an absorbent structure 16, intermediate the liquid pervious outer layer 12 and the liquid impervious outer layer 14, and provided in the embodiment of Figure 1 as discrete elements between the two outer layers. The layered continuous web-like layered structure can also comprise further layers and elements, as well as, alternatively or in combination, a continuous absorbent structure, as it is known in the art.

An adhesive application unit 36 applies a layer of pressure sensitive adhesive 38 in a preferably continuous stripe onto the liquid impervious outer layer 14 of the continuous web-like layered structure 34. Typically the adhesive application unit 30 is a hot melt coating unit, for example preferably a known bar coating apparatus, for the application of a layer of the preferred hot melt pressure sensitive adhesive onto the liquid impervious outer layer 14 in the desired thickness. Alternatively other known methods of adhesive application can be used, for example non-contact methods such as hot melt spraying. According to a preferred embodiment of the present invention, the pressure sensitive adhesive 38 is applied in a continuous stripe which is slightly narrower than the continuous web-like layered structure 34, as indicated in Figure 1, which actually represents a portion of the continuous web-like layered structure 34, subsequent the application of the pressure sensitive adhesive 38. The width of the continuous stripe of pressure sensitive adhesive 38 can be suitably tailored, such that for example, in the final individual disposable absorbent article 10 cut from the continuous web-like layered structure 34, the adhesive covers most of the liquid impervious outer layer 14, i.e. the backsheet, leaving two areas 42 at both transverse ends of the article free of adhesive; alternatively an adhesive free area can be provided at only one transverse end of the article 10, or in different locations along the perimeter 18, as can be readily determined by the man skilled in the art. An adhesive free area along the perimeter 18 advantageously provides the finished absorbent article 10 with a free edge, not attached to a substrate, i.e. respectively to the wrapper element before use, and to the undergarment during use. Said free edge can be easily grasped by a user in order to detach the article from the respective substrate. The maximum depth of this adhesive free area or areas 42 can be suitably selected, for example between 1 mm and 20 mm, preferably from 5 mm and 15 mm, more preferably being around 10 mm.

The continuous web-like layered structure 34 with the layer of pressure sensitive adhesive 38 applied thereon is conveyed to the cutting unit 44 comprising a cutting implement with at least one knife. Typically, as illustrated in Figure 4, the cutting unit 44 comprises a knife roll 46 and a counter roll 48 (anvil roll). The knife roll 46 comprises a series of knives 50 each having a shape actually corresponding to the perimeter along which the individual disposable absorbent articles are to be cut from the continuous web-like layered structure 34, hence ultimately corresponding to the outer perimeter 18 of the disposable absorbent articles 10. The knives 50 are intended to cut the continuous web-like layered structure 34 directly through the layer of pressure sensitive adhesive 38. In order to avoid adhesive build up and sticking on the cutting implement the knife is internally cooled to a temperature typically below the dew point in the working conditions. This results in the formation of water condensate on the surface of the knife blade which prevents any sticking of the adhesive onto the knife. Typically, in the embodiment illustrated in Figure 4, the entire knife roll 46 is internally cooled at the desired temperature.

The trimmed material 52, 52' resulting from the cutting step is then withdrawn and disposed of after cut. Typically, as shown in Figure 4, the two continuous trim stripes 52 are separated from the cutting unit 44 and disposed of with known means. The discrete trim material elements 52', created in the cutting step in the preferred embodiment illustrated in Figures 1 and 4, where the individual disposable absorbent articles 10 are made from the continuous web-like layered structure 34 being adjacent to one another at two points in correspondence of the wider transverse sections in order to save material, are instead preferably withdrawn within the counter roll 48 by e.g. a vacuum system, not shown in the drawing, and disposed of with known means. Different means for withdrawing and disposing of the trimmed material can be also provided, as can be readily determined by the skilled man, for example in case the individual disposable absorbent articles 10 are cut from the continuous web-like layered structure 34 in a different arrangement.

For example, if the individual disposable absorbent articles are not cut from the continuous web-like layered structure 34 being adjacent to one another, but instead with a small gap between each pair of subsequent articles, the amount of trimmed material will be slightly increased, but it will consist in a continuous, ladder-shaped element which can be easily withdrawn altogether from the cutting unit 44 and suitably disposed of, without the need of a separate device, e.g. a vacuum system, for withdrawal of the discrete trim material elements 52'as shown in Figure 4.

After the cutting step, the continuous layer of pressure sensitive adhesive 38 provides the adhesive elements 24 on the backsheet layer 14 of the individual disposable absorbent articles 10 cut from the continuous web-like layered structure 34. The adhesive element 24 extends along substantially the entire length of the longitudinal perimeter segments 20, as shown in Figures 1 and 4.

A distinct system, typically a vacuum system as well, is also provided in the counter roll 48 in order to keep after the cutting step the newly formed individual disposable absorbent articles 10 onto the outer surface of the counter roll 48 until they are transferred onto a continuous band of wrapper material 54 and releasably adhered thereto by means of the respective adhesive elements 24 at a coupling unit 56 comprising a pressing roll or bump roll 58 which presses the individual disposable absorbent articles 10 onto the wrapper material 54. The bump roll 58 can be pitched, as shown in Figure 4, or cylindrical with a smooth and preferably resilient and flexible outer surface.

As it is known in the art, the speed of the band of wrapper material 54, registered with the peripheral speed of the bump roll 58, can be suitably selected and is typically higher than the peripheral speed of the counter roll 48, such that subsequent individual disposable absorbent articles 10 are coupled with the desired spacing (pitch) onto the band of wrapper material 54.

The band of wrapper material 54 with attached individual disposable absorbent articles 10 subsequently proceeds to folding/cutting/sealing units, not illustrated in Figure 4, where the individually packaged disposable absorbent articles are formed. This can be done according to known techniques, as can be readily determined by the man skilled in the art. For example, in a preferred embodiment of the present invention, the band of wrapper material 54 with attached individual disposable absorbent articles 10 can proceed to a folding unit where the band 54 and the affixed articles 10 are continuously folded as a unit into thirds about two fold axes substantially parallel to the direction of travel of the band 54, corresponding to the axes 30 and 30' illustrated in Figure 2 with reference to a single article 10 releasably attached to its respective wrapper element 28. Individually packaged disposable absorbent articles are then provided by cutting and sealing the three folded band 54 comprising the individual articles 10 along spaced lines perpendicular to the direction of travel, which in turn correspond to the superimposed and sealed together longitudinal edges of each wrapper element 28, as shown for example in Figure 3 in a partially folded configuration. Of course the steps can be also performed in the reverse order, i.e. first cutting each wrapper element 28 with the respective attached individual disposable article 10, and then folding and sealing it in order to form the bag or pouch 26.

According to a preferred embodiment of the present invention, the counter roll 48 of the cutting unit 44 can also be cooled to prevent possible adhesive buildup onto the counter roll as well. The cooling of the knife, typically of the entire knife roll 46, and of the counter roll 48 can be achieved with known means, for example by means of circulation of a known cooling fluid, such as a water-glycol solution. Typically, and preferably, the whole knife roll 46, and optionally the whole counter roll 48 are uniformly cooled, although it is also possible that only selected portions of the rolls are cooled, for example the knives in the knife roll 46, or the areas corresponding to the individual disposable absorbent articles in the counter roll 48.

Actual temperatures at which the knife roll 46 and optionally the counter roll 48 shall be cooled can depend on the environmental conditions at which the cutting step occurs. Typically the room temperature is between 20°C and 30°C, preferably at about 25°C. The relative humidity is at least 40%, preferably between 40% and 60%, more preferably between 45% and 55%, most preferably about 50%. In such typical preferred process conditions, the temperature of the knife roll 46 is preferably kept between 0°C and 6°C, more preferably between 2°C and 4°C, most preferably at about 3°C. When the counter roll 48 is also preferably cooled, its temperature is kept between 5°C and 20°C, preferably between 7°C and 15°C, more preferably at about 10°C. It is particularly preferred, in order to prevent or limit formation of water condensate on areas of the counter roll 48 contacting the individual disposable absorbent articles 10 during their transport to the coupling unit 56, that the temperature of the counter roll, preferably also cooled as described above, be kept lower than the temperature of the knife roll 46, with a difference ( T) between 2°C and 20°C, preferably between 5°C and 10°C.

In a particularly preferred embodiment of the present invention, with typical room conditions of 24°C and 50% relative humidity, the temperature of the knife roll 46 is about 3°C, and the temperature of the counter roll 48 is about 10°C, with a difference T of about 7°C.

According to a further preferred embodiment of the present invention, the areas 60 of the knife roll 46 comprised within the perimeters defined by each knife 50 can be recessed with respect to the outer surface of the knife roll 46, in order to avoid accidental contact of the roll in said areas 60 with the adhesive 38, and to further reduce the risk of jamming of the apparatus, especially at the high speed which is typical of this type of processes. The depth of these recessed areas is preferably constant, and can be suitably tailored according to the thickness of the formed individual disposable absorbent articles 10, as can be readily determined by the skilled man.

The method and the apparatus of the present invention has been so far described in the arrangement where the individual disposable absorbent articles are cut from the continuous web-like layered structure 34 oriented in cross direction (CD), i.e. with the respective longitudinal axes perpendicular to the direction of travel of the web structure. Alternatively, the individual disposable absorbent articles can also be cut from the continuous web-like layered structure arranged in a machine direction (MD) orientation, and the respective changes and adjustments in the process and in the apparatus are readily at hand of the skilled man.

The adhesive layer applied in a preferably continuous stripe can have any preferred width or pattern, with a constant of variable basis weight. Preferably the application is in a continuous stripe having constant width and constant basis weight, as illustrated in the appended drawings. Preferred application method is hot melt bar coating, also known as slot coating. The adhesive layer can be applied preferably with a basis weight from 10 g/m² to 40 g/m², more preferably from 15 g/m² and 30 g/m², even more preferably from 20 g/m² and 25 g/m².

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A process for manufacturing individually packaged disposable absorbent articles comprising the steps of:
• providing a continuous web-like layered structure comprising at least a liquid pervious outer structure, a liquid impervious outer structure, and an absorbent material intermediate said liquid pervious outer structure and said liquid impervious outer structure,
• applying a layer of pressure sensitive adhesive for garment attachment onto at least part of said liquid impervious outer structure,
• cutting individual disposable absorbent articles along a perimeter from said continuous web-like layered structure, said cutting occurring at least partially directly through said layer of pressure sensitive adhesive, said perimeter of each of said individual disposable absorbent articles having longitudinal perimeter segments substantially parallel to a longitudinal axis of said individual disposable absorbent articles; wherein said cutting step is carried out with a cutting implement comprising at least one knife which is internally cooled;
• releasably attaching each of said individual disposable absorbent articles to a respective wrapper element by means of said layer of pressure sensitive adhesive;
• folding said wrapper element around each of said individual disposable absorbent articles;
• sealing said wrapper element around at least part of said perimeter of each of said individual disposable absorbent articles in order to form said individually packaged disposable absorbent articles.

2. A process according to claim 1, wherein said wrapper element has longitudinal side portions extending outward from said longitudinal perimeter segments of said individual disposable absorbent article.

3. A process according to claim 2, wherein each of said individual disposable absorbent articles and said respective wrapper elements are folded as a unit about at least two fold-axes substantially perpendicular to said longitudinal axis of said individual disposable absorbent articles, and said longitudinal side portions are frangibly sealed together to form said individually packaged disposable absorbent articles.

4. A process according to any preceding claim, wherein said wrapper elements are provided from a continuous band of wrapper material.

5. A process according to any preceding claim, wherein said layer of pressure sensitive adhesive is applied as a continuous stripe.

6. A process according to any preceding claim, wherein said layer of pressure sensitive adhesive is applied with a basis weight from 10 g/m² and 40 g/m², preferably from 15 g/m² and 30 g/m², more preferably from 20 g/m² and 25 g/m².

7. A process according to any preceding claim, wherein said cutting implement is comprised in a cutting unit comprising a knife roll and a counter roll, said knife roll being internally cooled and comprising said at least one knife.

8. A process according to any preceding claim, wherein said at least one knife is cooled at a temperature between 0°C and 6°C, preferably between 2°C and 4°C, most preferably at 3°C.

9. A process according to claim 7, wherein said counter roll is internally cooled.

10. A process according to claim 9, wherein said counter roll is cooled at a temperature between 5°C and 20°C, preferably between 7°C and 15°C, more preferably at 10°C.

11. A process according to claims 9 or 10, wherein the temperature of said counter roll is lower than the temperature of said at least one knife, with a difference T between 2°C and 20°C, preferably between 5°C and 10°C.

12. A process according to any of claims 7 to 11, wherein the area of said knife roll comprised within the perimeter defined by the at least one knife is recessed with respect to the outer surface of said knife roll.

13. An apparatus for manufacturing individually packaged disposable absorbent articles according to the process of any of the preceding claims, said apparatus comprising at least:
• a supply unit of said continuous web-like layered structure;
• a pressure sensitive adhesive application unit;
• a supply unit of said wrapper material;
• a cutting unit comprising a knife roll and a counter roll;
• a coupling unit for releasably attaching said individual disposable absorbent articles to said wrapper material;
• a folding/cutting/sealing unit for forming said individually packaged disposable absorbent article;
wherein said knife roll comprises at least one knife which is internally cooled.

## Patentansprüche

1. Verfahren zur Herstellung von einzeln verpackten Einweg-Absorptionsartikeln, folgende Schritte umfassend:
• Bereitstellen einer kontinuierlichen, bahnähnlichen Schichtstruktur, die mindestens eine flüssigkeitsdurchlässige äußere Struktur, eine flüssigkeitsundurchlässige äußere Struktur und ein Absorptionsmaterial zwischen der flüssigkeitsdurchlässigen äußeren Struktur und der flüssigkeitsundurchlässigen äußeren Struktur umfasst,
• Auftragen einer Schicht aus druckempfindlichem Klebstoff für die Befestigung von Kleidung an mindestens einem Teil der flüssigkeitsundurchlässigen äußeren Struktur,
• Abtrennen von einzelnen Einweg-Absorptionsartikeln entlang eines Umfangsrands von der kontinuierlichen, bahnähnlichen Struktur, wobei das Abtrennen zumindest teilweise direkt durch die Schicht aus druckempfindlichem Klebstoff hindurch erfolgt, wobei der Umfangsrand jedes der einzelnen Einweg-Absorptionsartikel Längsrandsegmente aufweist, die im Wesentlichen parallel zu einer Längsachse der einzelnen Einweg-Absorptionsartikel sind, wobei der Trennschritt mit einer Trenneinrichtung durchgeführt wird, die mindestens ein Messer aufweist, das intern gekühlt wird,
• lösbares Befestigen jedes der einzelnen Einweg-Absorptionsartikel an einem jeweiligen Hüllelement mittels der Schicht aus druckempfindlichem Klebstoff,
• Falten des Hüllelements um jedes der einzelnen Einweg-Absorptionsartikel,
• Siegeln des Hüllelements um zumindest einen Teil des Umfangsrands jedes der einzelnen Einweg-Absorptionsartikel, um die einzeln verpackten Einweg-Absorptionsartikel zu bilden.

2. Verfahren nach Anspruch 1, wobei das Hüllelement Längsseitenabschnitte aufweist, die von den Längsrandsegmenten des einzelnen Einweg-Absorptionsartikels nach außen verlaufen.

3. Verfahren nach Anspruch 2, wobei jeder der einzelnen Einweg-Absorptionsartikel und jedes der jeweiligen Hüllelemente als Einheit um mindestens zwei Faltachsen, die im Wesentlichen senkrecht zur Längachse der einzelnen Einweg-Absorptionsartikel sind, gefaltet werden und die Längsseitenabschnitte lösbar aneinander gesiegelt werden, um die einzeln verpackten Einweg-Absorptionsartikel zu bilden.

4. Verfahren nach einem der vorangehenden Ansprüche, wobei die Hüllelemente von einem kontinuierlichen Band aus Hüllmaterial bereitgestellt werden.

5. Verfahren nach einem der vorangehenden Ansprüche, wobei die Schicht aus druckempfindlichem Klebstoff als kontinuierlicher Streifen aufgebracht wird.

6. Verfahren nach einem der vorangehenden Ansprüche, wobei die Schicht aus druckempfindlichem Klebstoff mit einem Grundgewicht von 10 g/m² und 40 g/m², vorzugsweise von 15 g/m² und 30 g/m², bevorzugter von 20 g/m² und 25 g/m² aufgetragen wird.

7. Verfahren nach einem der vorangehenden Ansprüche, wobei die Trenneinrichtung aus einer Trenneinheit besteht, die eine Messerwalze und eine Gegenwalze umfasst, wobei die Messerwalze intern gekühlt wird und mindestens ein Messer umfasst.

8. Verfahren nach einem der vorangehenden Ansprüche, wobei das mindestens eine Messer bei einer Temperatur zwischen 0 °C und 6 °C, vorzugsweise zwischen 2 °C und 4 °C, am meisten bevorzugt bei 3 °C gekühlt wird.

9. Verfahren nach Anspruch 7, wobei die Gegenwalze intern gekühlt wird.

10. Verfahren nach Anspruch 9, wobei die Gegenwalze bei einer Temperatur zwischen 5 °C und 20 °C, vorzugsweise zwischen 7 °C und 15 °C, bevorzugter bei 10 °C gekühlt wird.

11. Verfahren nach einem der Ansprüche 9 oder 10, wobei die Temperatur der Gegenwalze niedriger ist als die Temperatur des mindestens einen Messers, wobei ein Unterschied ΔT zwischen 2 °C und 20 °C, vorzugsweise zwischen 5 °C und 10 °C liegt.

12. Verfahren nach einem der Ansprüche 7 bis 11, wobei die Fläche der Messerwalze, die in dem Umfangsrand enthalten ist, der von dem mindestens einen Messer definiert wird, in Bezug auf die Außenfläche der Messerwalze eingetieft ist.

13. Vorrichtung zur Herstellung von einzeln verpackten Einweg-Absorptionsartikeln gemäß dem Verfahren nach einem der vorangehenden Ansprüche, wobei die Vorrichtung zumindest aufweist:
• eine Zufuhreinheit für die kontinuierliche bahnartige Schichtstruktur,
• eine Auftragungseinheit für druckempfindlichen Klebstoff,
• eine Zufuhreinheit für das Hüllmaterial,
• eine Trenneinheit, die eine Messerwalze und eine Gegenwalze umfasst,
• eine Verbindungseinheit, die die einzelnen Einweg-Absorptionsartikel lösbar an dem Hüllmaterial befestigt,
• eine Falt-/Trenn-/Siegelungseinheit, die die einzeln verpackten Einweg-Absorptionsartikel bildet,
wobei die Messerwalze mindestens ein Messer umfasst, das intern gekühlt wird.

## Revendications

1. Procédé pour la fabrication d'articles absorbants jetables conditionnés individuellement comprenant les étapes consistant à :
• fournir une structure en couche de type nappe continue comprenant au moins une structure externe perméable aux liquides, une structure externe imperméable aux liquides, et un matériau absorbant intermédiaire entre ladite structure externe perméable aux liquides et ladite structure externe imperméable aux liquides,
• appliquer une couche d'adhésif sensible à la pression pour attachement à un vêtement sur au moins une partie de ladite structure externe imperméable aux liquides,
• couper des articles absorbants jetables individuels le long d'un périmètre de ladite structure en couche de type nappe continue, ladite découpe se produisant au moins partiellement directement à travers ladite couche d'adhésif sensible à la pression, ledit périmètre de chacun desdits articles absorbants jetables individuels ayant des segments de périmètre longitudinaux sensiblement parallèles à un axe longitudinal desdits articles absorbants jetables individuels ; dans lequel ladite étape de découpe est effectuée avec un instrument de découpe comprenant au moins un couteau qui est refroidi de façon interne ;
• fixer de façon libérable chacun desdits articles absorbants jetables individuels à un élément d'emballage respectif au moyen de ladite couche d'adhésif sensible à la pression ;
• plier ledit élément d'emballage autour de chacun desdits articles absorbants jetables individuels ;
• sceller ledit élément d'emballage autour d'au moins une partie dudit périmètre de chacun desdits articles absorbants jetables individuels afin de former lesdits articles absorbants jetables conditionnés individuellement.

2. Procédé selon la revendication 1, dans lequel ledit élément d'emballage a des parties latérales longitudinales s'étendant vers l'extérieur à partir desdits segments de périmètre longitudinaux dudit article absorbant jetable individuel.

3. Procédé selon la revendication 2, dans lequel chacun desdits articles absorbants jetables individuels et lesdits éléments d'emballage respectifs sont pliés en tant qu'une unité autour d'au moins deux axes de pliage essentiellement perpendiculaires audit axe longitudinal desdits articles absorbants jetables individuels, et lesdites parties latérales longitudinales sont scellées de façon frangible conjointement de façon à former lesdits articles absorbants jetables conditionnés individuellement.

4. Procédé selon l'une quelconque des revendications précédentes, dans lequel lesdits éléments d'emballage sont fournis à partir d'une bande continue de matériau "d'emballage.

5. Procédé selon l'une quelconque des revendications précédentes, dans lequel ladite couche d'adhésif sensible à la pression est appliquée en tant que bande continue.

6. Procédé selon l'une quelconque des revendications précédentes, dans lequel ladite couche d'adhésif sensible à la pression est appliquée avec une masse surfacique de 10 g/m² et 40 g/m², de préférence de 15 g/m² et 30 g/m², plus préférablement de 20 g/m² et 25 g/m².

7. Procédé selon l'une quelconque des revendications précédentes, dans lequel ledit instrument de découpe est compris dans une unité de découpe comprenant un rouleau de découpe et un contre-rouleau, ledit rouleau de découpe étant refroidi de façon interne et comprenant ledit au moins un couteau.

8. Procédé selon l'une quelconque des revendications précédentes, dans lequel ledit au moins un couteau est refroidi à une température entre 0 °C et 6 °C, de préférence entre 2 °C et 4 °C, le plus préférablement à 3 °C.

9. Procédé selon la revendication 7, dans lequel ledit contre-rouleau est refroidi de façon interne.

10. Procédé selon la revendication 9, dans lequel ledit contre-rouleau est refroidi à une température entre 5 °C et 20 °C, de préférence entre 7 °C et 15 °C, plus préférablement à 10 °C.

11. Procédé selon les revendications 9 ou 10, dans lequel la température dudit contre-rouleau est inférieure à la température dudit au moins un couteau, avec une différence ΔT entre 2 °C et 20 °C, de préférence entre 5 °C et 10 °C.

12. Procédé selon l'une quelconque des revendications 7 à 11, dans lequel la zone dudit rouleau de découpe comprise au sein du périmètre défini par l'au moins un couteau est renfoncée par rapport à la surface externe dudit rouleau de découpe.

13. Appareil pour la fabrication d'articles absorbants jetables conditionnés individuellement selon le procédé selon l'une quelconque des revendications précédentes, ledit appareil comprenant au moins :
• une unité d'alimentation de ladite structure en couche de type nappe continue ;
• une unité d'application d'adhésif sensible à la pression ;
• une unité d'alimentation dudit matériau d'emballage ;
• une unité de découpe comprenant un rouleau de découpe et un contre-rouleau ;
• une unité de couplage pour fixer de façon libérable lesdits articles absorbants jetables individuels audit matériau d'emballage ;
• une unité de pliage/découpe/scellage pour former ledit article absorbant jetable conditionné individuellement ;
dans lequel ledit rouleau de découpe comprend au moins un couteau qui est refroidi de façon interne.
